# EUROPEAN PATENT APPLICATION

(11) **EP 4 813 970 A1**
(43) Date of publication of application: **30.09.2026**
(21) Application number: 24894507.3
(22) Date of filing: 14.11.2024
(51) Int. Cl.: C07C 227/40, C07C 229/22, A61K 31/198, A61P 25/00, A61P 3/00, A61P 1/16, A61P 9/00, A23L 33/175

(54) **L-SERINE HAVING REDUCED HEAVY METAL AMOUNT, PHARMACEUTICAL COMPOSITION THEREOF, PREPARATION METHOD THEREFOR, AND USE THEREOF**

(30) Priority: 20.11.2023 KR 20230160923
(71) Applicant: Astrogen, Inc., Daegu 41072 (KR)
(72) Inventor: HWANG, Su-Kyeong, Daegu 41072 (KR); RYU, Hyung Chul, Daegu 41072 (KR)
(74) Representative: Dehns
(86) International application number: PCT/KR2024/017938
(87) International publication number: WO 2025/110614

(57) **Abstract**

The present invention relates to L-serine having a reduced heavy metal amount, a pharmaceutical composition thereof, a preparation method therefor, and a use thereof. The L-serine having a reduced heavy metal amount, according to the present invention, can be used for preventing, alleviating, or treating neurological diseases or metabolic diseases.

## Description

### Technical Field

The present invention relates to L-serine with reduced heavy metal content, a pharmaceutical composition, a preparation method, and a use thereof.

### Background Art

Heavy metals, which are not biodegradable, are heavy metals having a specific gravity of 4.0 or more from the chemical aspect, in which iron, zinc, copper, *etc*. are substances necessary for the human body, whereas lead, mercury, cadmium, arsenic, *etc*. are not only harmful to living organisms, but can be accumulated in the body without being metabolized and cause various serious diseases, including cancer. In particular, lead, mercury, cadmium, arsenic, *etc*. cause various adverse effects on systemic metabolic activities, including the central nervous system, peripheral neurons, circulatory system, lungs, kidneys, liver, blood, digestive system, reproductive system, *etc*. In particular, when the nervous system is exposed to harmful heavy metals, it may lead to serious conditions (*e.g*., degenerative neurological diseases, *etc*.) from which recovery is impossible, and fetuses and children are more vulnerable to the exposure to heavy metals, neurodevelopmental processes may be significantly affected, and thus people may have to live with developmental disabilities or psychiatric/neurological symptoms throughout their lives. Thus far, the correlation between heavy metals, especially class 1 heavy metals (*e.g*., lead, mercury, cadmium, arsenic, *etc*.), and neurological and metabolic diseases has been studied and reported.

It was observed in literature articles [J Child Neurol, 2002, 19, 692-695, Clin Chem 1981, 27, 879-881] that cadmium and lead were significantly higher in children with autism spectrum disorder than in children with normal development, as a result of measuring the contents of harmful heavy metals in the hair of normally developing children and children with autism spectrum disorder.

It was confirmed in the literature article [Journal of Trace Elements in Medicine and Biology, 2021] that by studying the effects of neurotoxic metals (lead, mercury, cadmium, nickel, and manganese) as environmental factors on autism spectrum disorder, children have immature and inefficient metabolic and detoxification processes, and particularly, infants are more vulnerable because their immune systems are weak and cannot effectively eliminate toxic substances, and thus autism symptoms could be alleviated by the use of anti-heavy metal treatment.

L-serine, which is a non-essential amino acid, is endogenous and used in a variety of ways, including foods, health functional foods, food additives, and drugs for intravenous injection. The present inventors conducted studies on a health functional food composition for preventing or mitigating neurological diseases containing L-serine as an active ingredient through Korean Patent Application No. 2019-0046017, and then, confirmed through a phase 2 clinical trial that when the composition was administered to children under 11 years of age at a dose of 400 mg/kg for 24 weeks, the treatment efficiency was about 40% without any unusual side effects. It was confirmed from the clinical results conducted by the present inventors that the therapeutic effect could be maximized when L-serine was administered in high doses for a long period of time.

In other clinical trials involving humans, as a result of long-term administration of L-serine at a dose of 400 mg/kg/day for up to 2 years in patients with hereditary sensory and autonomic neuropathy type 1 (HSAN1), there were cases where the effects of improving safety and Charcot-Marie-Tooth Neuropathy Score (CMTNS) were confirmed, and it was reported that the oral administration of L-serine twice a day at a maximum dose of 15 g/day for 6 months to patients with amyotrophic lateral sclerosis (ALS) was safe and showed the effect of delaying the progression of the patients' disease. Additionally, in a literature where L-serine was administered orally to HSAN1 patients at a dose of up to 400 mg/kg/day for 10 and 52 weeks, it was confirmed that 1-deoxySL (dSL), which causes neurodegeneration, was reduced by L-serine, thereby lowering the level of neurotoxicity. Further, there were other reports that even for ALS patients in phase 2 clinical trials, L-serine was shown to be safe in ALS patients at very high doses of up to 15 g twice a day for 6 months; in a test where the efficacy and adverse reactions of L-serine in pregnant women and children with the genetic factor for congenital microcephaly were observed, maternal supply of L-serine during pregnancy was shown to improve brain growth in fetuses with 3-PGDH deficiency, and the administration of L-serine to postnatal children (birth to 4 years old) was shown to prevent neurological symptoms, and additionally, no adverse reactions were observed in pregnant women, fetuses, and newborns.

U.S. Patent Publication No. 2018/0027781 discloses a method for treating neurometabolic diseases, such as Alzheimer's disease, Parkinson's disease, amyotrophic lateral sclerosis (ALS), progressive supranuclear palsy (PSP), lewy body dementia (LBD), amyotrophic lateral sclerosis/parkinsonism dementia complex (ALS/PDC), Huntington's disease (HD), Pick's disease, and frontotemporal dementia (FTD), by the administration of L-serine at a dose of 750 mg/kg to 1,000 mg/kg per day.

Korean Patent Publication No. 10-2022-0124007 discloses a pharmaceutical composition for preventing or treating liver fibrosis containing L-serine as an active ingredient, and that L-serine can be used in combination with losartan to prevent and treat liver disease.

However, when L-serine is used as an active ingredient for medical drugs or health functional foods, the use of L-serine, which is controlled within a simple daily allowable limit, may reduce the original drug efficacy due to the nature of heavy metals being accumulated without biodegradation resulting from long-term use in high doses, and particularly, it may cause serious illness in children and infants. In particular, it is necessary that L-serine intended for use in subjects with neurological diseases or metabolic diseases be controlled to be lower than the standard allowable level for class 1 heavy metals.

For example, when applying the EU heavy metal limit for omega 3 (Table 1), which is widely known as a health functional food, to a dose of L-serine used in phase 2 clinical trials for treating patients with autism spectrum disorder, in the case of the administration of L-serine to a 40 kg autism spectrum disorder patient aged 2-11 years for 24 weeks, the amount of each class 1 heavy metal per 16 g of daily administration of L-serine corresponds to 1.6 µg, and the maximum amount of class 1 heavy metals that can accumulate over 24 weeks corresponds to 1,152 µg, thus indicating that a significant amount of heavy metals can accumulate in the body. Moreover, considering even the routes through which class 1 heavy metals can be ingested through foods, drinking water, and beverages, the importance of controlling these class 1 heavy metals is even more urgent.

**[Table 1]**

| EU standards of allowable level 1 heavy metal content for omega-3 | |
|---|---|
| Heavy Metals | Content |
| Arsenic (As) | 0.1 (µg/g) |
| Lead (Pb) | 0.1 (µg/g) |
| Cadmium (Cd) | 0.1 (µg/g) |
| Mercury (Hg) | 0.1 (µg/g) |

The calculated daily heavy metal exposure amount for L-serine is consistent with the permitted daily exposure (PDE) of class 1 heavy metals of concern for toxicity recommended by the Ministry of Food and Drug Safety (Korea) shown in Table 2 below. However, considering that L-serine is being used as a drug or health functional food in high doses for a long period of time, in its effective dose, an extremely large amount of class 1 heavy metals will accumulate in the body thereby affecting the body.

**[Table 2]**

| Allowable daily exposure to metal impurities | | | | |
|---|---|---|---|---|
| Metals | Classification (grade) | Oral Administration of PDE (µg/day) | Parenteral Administration of PDE (µg/day) | Inhalation of PDE (µg/day) |
| Cadmium (Cd) | 1 | 5 | 2 | 2 |
| Lead (Pb) | 1 | 5 | 5 | 5 |
| Arsenic (As) | 1 | 15 | 15 | 2 |
| Mercury (Hg) | 1 | 30 | 3 | 1 |

Accordingly, the present inventors have confirmed various methods for removing heavy metals from L-serine; and as a result, they have confirmed that theoretically, a method of precipitation in the form of hydroxide or sulfide, an ion exchange method, an adsorption treatment method using activated carbon, an electrochemical treatment method, a solvent extraction method, an evaporation method, *etc*., which are methods frequently used to remove heavy metals, have disadvantages in that these methods are expensive to carry out industrially, may incompletely remove heavy metals depending on the items, products are restricted by pH conditions, limited to high concentrations, or produce harmful sludge or by-products.

Specifically, precipitation with sulfide or hydroxide carries risks such as heat generation when used in large-quantity purification methods. In particular, L-serine, which has a chiral carbon, has a risk of switching optical activity due to heat generation under strong acid or base conditions, problems occur, after removing heavy metals, such as damage to equipment and reduced yield due to hygroscopicity during filtration, and additional costs arise due to expenses during the neutralization process and wastewater treatment. Meanwhile, when L-serine is dissolved in water and stirred with an ion exchange resin, it has problems in that the water solubility of L-serine decreases thus requiring a large amount of purified water, precipitation occurs during the ion exchange at the area where the ion exchange resin and L-serine meet, which not only reduces the effect of ion exchange, but also makes the recycling of ion exchange resin difficult. Meanwhile, activated carbon is rarely used industrially and stirred with purified products due to the increased risk of contamination of the reactor and leakage during filtration, and in order to prevent a decrease in yield, it requires to go through with a process of removing the dissolved solvent once again, which takes a large amount of time and money.

Therefore, the present inventors have confirmed that it is possible to obtain a primary heavy metal removal effect by maximizing the water solubility of existing L-serine through appropriate pH adjustment in the solution, it is possible to significantly reduce heavy metals compared to the existing L-serine merely by allowing L-serine to pass through activated carbon and/or ion exchange resin, and it is possible to obtain the desired L-serine as a solid precipitate merely by adjusting the pH in a solution phase, and thus a significant cost-reducing effect can be expected.

As such, the present inventors have developed a method for economically preparing and/or purifying L-serine with reduced content of class 1 heavy metals for use in the prevention, treatment, or improvement of neurological diseases or metabolic diseases.

### Disclosure of Invention

### Technical Problem

An object of the present invention is to provide L-serine with reduced heavy metal content which can increase drug efficacy and reduce side effects compared to existing L-serine when used to prevent, improve, or treat neurological diseases or metabolic diseases.

### Solution to Problem

In order to achieve the above object, the present invention provides L-serine of the following Formula 1 with reduced heavy metal content.

The present invention also provides a pharmaceutical composition for preventing or treating neurological diseases or metabolic diseases including the L-serine.

The present invention also provides a health functional food for preventing or mitigating neurological diseases or metabolic diseases, which includes the L-serine as an active ingredient.

The present invention also provides a method for reducing heavy metal content in L-serine, which includes the following steps:
(a) dissolving L-serine of Formula 1 below in water;
(b) adjusting the pH of the aqueous solution of L-serine obtained in the step (a) with an acid;
(c) passing the pH-adjusted aqueous solution of L-serine through activated carbon and/or ion exchange resin; and
(d) neutralizing the aqueous solution of L-serine obtained in the step (c) while gradually lowering the temperature, 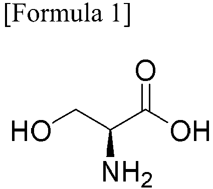 wherein the heavy metal is one or more selected from the group consisting of cadmium, arsenic, lead, and mercury.

### Advantageous Effects of Invention

The present invention can maximize the effect of L-serine, which can prevent and treat diseases selected from the group consisting of developmental disorders, developmental delays, autism spectrum disorder, attention deficit hyperactivity disorder (ADHD), Alzheimer's disease, Parkinson's disease, Huntington's disease, emotional disorders, sleep disorders, and movement disorders, and which can be used for preventing and treating diseases selected from the group consisting of fatty liver, nonalcoholic steatohepatitis, abdominal obesity, hypertriglyceridemia, hypo-HDL (high density lipoprotein)-cholesterolemia, hypertension, abnormal glucose metabolism syndrome, type 2 diabetes, cardiovascular disease, myocardial infarction, and angina pectoris, and can minimize the side effects of class 1 heavy metals that may accumulate due to long-term high dose administration of L-serine.

### Brief Description of Drawings

Fig. 1 is a graph confirming the inhibitory effect of the L-serine obtained in Example 1 of the present invention on mitochondrial damage depending on the difference in the content of class 1 heavy metals in the L-serine.
Fig. 2 is a graph confirming the inhibitory effect of long-term administration of 9 months of the L-serine obtained in Example 1of the present invention and unpurified L-serine on animal survival in a model of Alzheimer's disease.
Fig. 3 shows images confirming the protective effect of drugs on neurotoxicity of the L-serine obtained in Example 1of the present invention and unpurified L-serine.

### Best Mode for Carrying out the Invention

Unless otherwise defined, all technical and scientific terms used in this specification have the same meaning as commonly understood by those skilled in the art to which the present invention pertains. In general, the nomenclature used herein is well known and commonly used in the art.

In one aspect, the present invention relates to a method for preparing L-serine with reduced heavy metal content.

An embodiment of the present invention relates to a method for preparing L-serine with reduced heavy metal content, which includes:
(a) dissolving L-serine of Formula 1 below in water;
(b) adjusting the pH of the aqueous solution of L-serine obtained in the step (a) with an acid;
(c) passing the pH-adjusted aqueous solution of L-serine through activated carbon and/or ion exchange resin; and
(d) neutralizing the aqueous solution of L-serine obtained in the step (c) while gradually lowering the temperature, 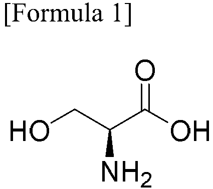 wherein the heavy metal is one or more selected from the group consisting of cadmium, arsenic, lead and mercury.

In the present invention, "L-serine", which is a type of amino acid constituting a protein and a monoamide of glutamic acid, refers to an L-amino acid having the structure of Formula 1 below.

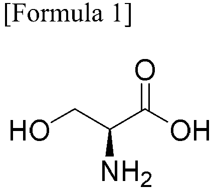

In the present invention, the step (a) is a step where L-serine is dissolved in water.

The amount of water used in the step (a) may be about 6-30 times the weight of L-serine used, preferably about 5-20 times, and most preferably about 4-10 times, but is not limited thereto. When water is used too much, the handling during the process becomes difficult and the amount of waste water discarded after the process increases; therefore, it is important to dissolve L-serine in a minimum amount of water to ensure economic efficiency. Additionally, when the temperature is raised, some L-serine is converted to D-Serine; therefore, it is necessary not to raise the temperature above room temperature.

In the present invention, the step (b) is a step in which the pH of the aqueous solution of L-serine obtained in the step (a) is adjusted with an acid.

When the pH of the aqueous solution of L-serine is adjusted to be acidic, not only the solubility of L-serine in water increases by more than 5 times compared to neutral L-serine thereby significantly reducing the process volume, but also increases the water solubility of heavy metals, which are difficult to remove due to their low water solubility in neutral pH, thereby increasing the removal rate of heavy metals. That is, the removal rate of heavy metals can be increased by increasing the water solubility of existing L-serine through appropriate pH adjustment in the solution.

In the step (b), the pH is adjusted with an acid, and the adjusted pH may be about pH 1.0 to about pH 5.0, preferably about pH 2.0 to about pH 4.5, and most preferably about pH 2.5 to about pH 4.0, but is not limited thereto. Meanwhile, when the pH is too low, the amount of the acid used increases, which reduces economic efficiency, and there is a risk of corrosion of equipment used in the process, such as reactors and filters that are made of metal and thus are weak to acids, and a risk of introduction of additional heavy metals, and in the subsequent neutralization process, a large amount of a basic solution is also required, thereby significantly reducing the economic feasibility of the process.

In the present invention, as the acid to be used, those known in the art may be selected and used as long as they do not deviate from the purpose of the present invention. Specifically, the acid may be one or more selected from the group consisting of hydrochloric acid, acetic acid, nitric acid, *p*-toluenesulfonic acid, fumaric acid, citric acid, succinic acid, salicylic acid, oxalic acid, bromic acid, phosphoric acid, methanesulfonic acid, tartaric acid, sulfuric acid, and mandelic acid, and preferably the acid may be hydrochloric acid, but is not limited thereto.

The acid may be used by adding it directly to a solution or by dissolving it in a certain amount of water.

In the present invention, the step (c) is a step where the pH-adjusted aqueous solution of L-serine is passed through activated carbon and/or ion exchange resin.

In the present invention, the activated carbon may be amorphous, cylindrical, or spherical with a density (g/cm³) of about 0.2-2.5, and amorphous activated carbon is most preferred, but is not limited thereto. The weight of activated carbon may be about 1/100 to about 1/10 of the weight of L-serine to be purified, preferably about 2/100 to about 5/100.

In the present invention, as an ion exchange resin, all of strongly acidic, weakly acidic, weakly basic, and strongly basic exchange resins may be used. The weight of the ion exchange resin is about 1-10 times the weight of L-serine to be purified, preferably about 2-8 times, and most preferably about 3-5 times the weight of the ion exchange resin, but is not limited thereto. For example, the solution may be passed through activated carbon and/or ion exchange resin or stirred together with activated carbon and/or ion exchange resin. When passing through activated carbon and/or ion exchange resin, a Nutche or pressure filter with an appropriate diameter may be used so that the solution containing dissolved L-serine can pass through while sufficiently contacting with the same. After use, the ion exchange resin may be recovered by filtration, washed with purified water, then dried, and reused.

In the present invention, in the step (c), either activated carbon or ion exchange resin may be used, or both activated carbon and ion exchange resin may be used. When the heavy metal content exceeds 50 ppb after the treatment with activated carbon, ion exchange resin may be used as a secondary purification to remove heavy metals. When activated carbon and ion exchange resin are used sequentially, it is preferable to use activated carbon first.

In the present invention, the step (d) is a step where the aqueous solution of L-serine obtained in the step (c) is neutralized while gradually lowering the temperature.

In the step (d), neutralization heat is generated due to neutralization, in which when the temperature is too high, some of the L-serine would not be in the same as in the original crystal form and discoloration may occur. Accordingly, the reaction should be performed at a temperature as low as possible, preferably at room temperature or lower, and most preferably at 15°C or lower, but the reaction temperature is not limited thereto. For example, the aqueous solution of L-serine may be neutralized by gradually lowering the temperature to about 10 to about 15°C.

In the present invention, as the base, those known in the art may be selected and used as long as they do not deviate from the purpose of the present invention. Specifically, the base may be one or more selected from the group consisting of potassium hydroxide, calcium hydroxide, potassium carbonate, calcium carbonate, sodium hydroxide, sodium carbonate, sodium phosphate, and potassium phosphate, most preferably sodium hydroxide and potassium hydroxide, but is limited thereto.

The base may be used in an amount equivalent to the acid used in the step (b), and the base may be added directly to the solution or dissolved in a certain amount of water.

In the present invention, after the step (d), a step (e) of performing filtration under reduced pressure and drying the obtained solid may be further included.

Specifically, in the step (e), all the filtration methods available in industrial production methods such as filtration under reduced pressure, centrifugation, and filter dryer are possible, and the drying temperature is about 20-80°C, preferably about 30-70°C, and most preferably, vacuum or hot air drying at 40-60°C may be used, but the filtration methods are not limited thereto. However, caution should be taken because discoloration may occur when the drying temperature is too high.

In the present invention, "heavy metal" or "class 1 heavy metal" is selected from the group consisting of cadmium, arsenic, lead, and mercury.

According to the method of the present invention, the content of heavy metals may be reduced to about 76 ppb or less, 70 ppb or less, 65 ppb or less, 60 ppb or less, or 55 ppb or less per gram of L-serine, respectively, and preferably reduced to about 50 ppb or less.

In still another aspect, the present invention relates to L-serine in which the contents of cadmium, arsenic, lead, and mercury are each 50 ppb or less per gram of L-serine.

The L-serine prepared according to the method of the present invention may be L-serine with a content of cadmium, arsenic, lead, and mercury of 50 ppb or less per gram of L-serine.

In still another aspect, the present invention relates to a pharmaceutical composition for the prevention or treatment of neurological diseases or metabolic diseases, which includes L-serine as an active ingredient with a content of cadmium, arsenic, lead, and mercury of 50 ppb or less per gram of L-serine.

As used herein, the term "treatment" refers to any action in which the symptoms of a disease are improved or beneficially changed by administering the composition according to the present invention, the term "prevention" refers to any action that inhibits or delays a disease by administering the composition according to the present invention, and the term "improvement" refers to any action that improves the bad condition of a disease by administering or ingesting the composition of the present invention to an individual.

The subject disease of the present invention for "treatment," "prevention," or "improvement" may be a neurological disease or metabolic disease. Specifically, the neurological diseases may be selected from the group consisting of developmental disorders, developmental delays, autism spectrum disorder, attention deficit hyperactivity disorder (ADHD), Alzheimer's disease, Parkinson's disease, Huntington's disease, emotional disorders, sleep disorders, and movement disorders; and the metabolic disease may be selected from the group consisting of fatty liver, nonalcoholic steatohepatitis, abdominal obesity, hypertriglyceridemia, hypo-HDL-cholesterolemia, hypertension, abnormal glucose metabolism syndrome, type 2 diabetes, cardiovascular disease, myocardial infarction, and angina pectoris.

The pharmaceutical composition of the present invention may be administered to a subject or individual by any administration route, and it may be a pharmaceutical composition for oral administration or injection. Preferably, the pharmaceutical composition of the present invention may be administered orally or intravenously.

The pharmaceutical composition of the present invention may further include a pharmaceutically acceptable carrier. As the pharmaceutically acceptable carrier, for example, a carrier for oral administration may be used. The carriers for oral administration may include lactose, starch, cellulose derivatives, magnesium stearate, stearic acid, *etc*. Additionally, the carriers for parenteral administration may include water, suitable oil, saline, aqueous glucose, glycol, *etc*., and may further include stabilizers, preservatives, *etc*. Suitable stabilizers include antioxidants such as sodium bisulfite, sodium sulfite, or ascorbic acid. Suitable preservatives include benzalkonium chloride, methyl- or propyl-paraben, and chlorobutanol. Other pharmaceutically acceptable carriers known in the art may be selected and used.

In addition to the above-identified ingredients, the pharmaceutical composition of the present invention may further include lubricants, wetting agents, sweeteners, flavoring agents, emulsifiers, suspending agents, preservatives, *etc*.

An effective dose may generally be about 50 mg/kg to about 1,000 mg/kg of body weight/day, preferably about 100 mg/kg to about 800 mg/kg of body weight/day, and most preferably, 200 mg/kg to 400 mg/kg of body weight/day in single or divided doses. Depending on age, species, and disease or condition to be treated, the administration level below the lower limit of the above-identified range may be appropriate. Higher doses may be used as long as there are no harmful side effects. Higher doses may be divided into several smaller doses for administration throughout the day. Appropriate doses may be determined using methods known in the art.

In the present invention, the content of L-serine in the pharmaceutical composition of the present invention may be about 60 wt% to about 100 wt%.

In still another aspect, the present invention relates to a health functional food composition for preventing or mitigating neurological diseases or metabolic diseases, which includes L-serine with a content of cadmium, arsenic, lead, and mercury of 50 ppb or less per gram of L-serine as an active ingredient.

The health functional food according to the present invention may be used with other foods or food additives in addition to the L-serine as an active ingredient, and may be used appropriately according to conventional methods.

In the present invention, the mixing amount of the active ingredient can be appropriately determined depending on the purpose of use, for example, prevention, health, or therapeutic treatment. For example, the content of L-serine in the health functional food composition of the present invention may be about 60 wt% to about 100 wt%.

The effective dose of the compound contained in the health functional food may be used in accordance with the effective dose of the therapeutic agent; however, in the case of long-term intake for health and hygiene purposes or health control, it may be below the above-identified range. Additionally, the compound may be used in amounts exceeding the above-identified range as long as there is no problem in terms of safety.

There are no special restrictions on the types of health functional foods, for example, the health functional foods may include, meats, sausages, bread, chocolate, candies, snacks, confectioneries, pizza, ramen, other noodles, gums, dairy products including ice creams, various soups, beverages, teas, drinks, alcoholic beverages, and vitamin complexes, but the health functional foods are not limited thereto.

The contents that overlap with the pharmaceutical composition of the present invention described above will be omitted.

In still another aspect, the present invention relates to a method for preventing or treating a neurological disease or metabolic disease in a subject, which includes administering to the subject L-serine having a cadmium, arsenic, lead, and mercury content of 50 ppb or less per gram of L-serine.

In the present invention, the term "subject" is used synonymously with an individual, and the subject may be a mammal, for example, a human, mouse, cow, horse, pig, dog, sheep, goat, or cat.

The contents that overlap with the pharmaceutical composition of the present invention described above will be omitted.

In still another aspect, the present invention relates to a use of L-serine, wherein the contents of cadmium, arsenic, lead, and mercury are each 50 ppb or less per gram of L-serine, for preventing or treating neurological diseases or metabolic diseases.

The contents that overlap with the pharmaceutical composition of the present invention described above will be omitted.

In still another aspect, the present invention relates to a use of L-serine, wherein the contents of cadmium, arsenic, lead, and mercury are each 50 ppb or less per gram of L-serine, in the preparation of a drug for preventing or treating neurological diseases or metabolic diseases.

The contents that overlap with the pharmaceutical composition of the present invention described above will be omitted.

Hereinafter, preferred embodiments are presented to aid understanding of the present invention. However, the following examples are merely illustrative of the present invention, and it is clear to those skilled in the art that various changes and modifications are possible within the scope and spirit of the present invention and it is also natural that such variations and modifications fall within the scope of the accompanying patent claims.

### Mode for the Invention

### Example

### Example 1

At room temperature, 4.0 kg of L-serine (Hunan Furui, China) and 20 L of purified water were added to a reactor and the mixture was stirred for 10 minutes. The pH of the mixture was adjusted to about 3.3 to about 3.5 using concentrated hydrochloric acid (Daejung Chemicals & Metals) and the complete dissolution was confirmed. After stirring for 1 hour, a celite pad was spread, and 150 g of amorphous activated carbon (Samchun Chemicals) was spread on top of the celite pad, and filtration was performed under reduced pressure, taking care not to damage the layered surface. The content of class 1 heavy metals in the filtered solution was checked using an ICP analyzer. A celite pad was spread and 12 kg of TRILITE^{®} CLR-10 (purchased from Samyang Corporation) was filled on top of the celite, and then the thus-obtained solution was added and filtered under reduced pressure. The ion exchange resin was collected, and the temperature of the thus-obtained solution was lowered to 10-15°C and its pH was adjusted to 6 to 7 with 6 N NaOH, was slowly stirred at 20-40 rpm for 3 hours, and filtration was performed under reduced pressure, and the thus-obtained solid was washed with 2 L of purified water. The resultant was dried under vacuum at 50°C to obtain L-serine with a yield of 91%.

**[Table 3]**

| Grade 1 heavy metal content in Example 1 | | | |
|---|---|---|---|
| | Before purification (a) | After purification with activated carbon (primary, b) | After purification with ion exchange resin (secondary, c) |
| Mercury content in L-serine | 72 ppb | 46 ppb | 39 ppb |
| Lead content in L-serine | 86 ppb | 56 ppb | 27 ppb |
| Cadmium content in L-serine | 69 ppb | 30 ppb | 22 ppb |
| Arsenic content in L-serine | 76 ppb | 51 ppb | 13 ppb |

### Example 2

At room temperature, 4.0 kg of L-serine (Hunan Furui, China) and 20 L of purified water were added to a reactor and the mixture was stirred for 10 minutes. The pH of the mixture was adjusted to about 3.3 to about 3.5 using concentrated hydrochloric acid (Daejung Chemicals & Metals) and the complete dissolution was confirmed. After stirring for 1 hour, a celite pad was spread, and 120 g of amorphous activated carbon (Samchun Chemicals) was spread on top of the celite pad, and filtration was performed under reduced pressure, being careful not to damage the layered surface. The content of class 1 heavy metals in the filtered solution was checked using an ICP analyzer. A celite pad was spread and 10 kg of TRILITE^{®} CLR-10 (purchased from Samyang Corporation) was filled on top of the celite, and then the thus-obtained solution was added and filtered under reduced pressure. The ion exchange resin was collected, and the temperature of the thus-obtained solution was lowered to 10-15°C and its pH was adjusted to 6 to 7 with 6 N NaOH, was slowly stirred at 20-40 rpm for 3 hours, and filtration was performed under reduced pressure, and the thus-obtained solid was washed with 2 L of purified water. The resultant was dried under vacuum at 50°C to obtain L-serine with a yield of 88%.

**[Table 4]**

| Grade 1 heavy metal content in Example 2 | | | |
|---|---|---|---|
| | Before purification (a) | After purification with activated carbon (primary, b) | After purification with ion exchange resin (secondary, c) |
| Mercury content in L-serine | 72 ppb | 55 ppb | 39 ppb |
| Lead content in L-serine | 86 ppb | 66 ppb | 47 ppb |
| Cadmium content in L-serine | 69 ppb | 37 ppb | 42 ppb |
| Arsenic content in L-serine | 76 ppb | 50 ppb | 18 ppb |

### Example 3

At room temperature, 4.0 kg of L-serine (Hunan Furui, China) and 20 L of purified water were added to a reactor and the mixture was stirred for 10 minutes. The pH of the mixture was adjusted to about 3.3 to about 3.5 using concentrated hydrochloric acid (Daejung Chemicals & Metals) and the complete dissolution was confirmed. After stirring for 1 hour, a celite pad was spread, and 150 g of amorphous activated carbon (Samchun Chemicals) was spread on top of the celite pad, and filtration was performed under reduced pressure, taking care not to damage the layered surface. The content of class 1 heavy metals in the filtered solution was checked using an ICP analyzer. A celite pad was spread and 12 kg of TRILITE^{®} CLR-8 (purchased from Samyang Corporation) was filled on top of the celite, and then the thus-obtained solution was added and filtration was performed under reduced pressure. The ion exchange resin was collected, and the temperature of the thus-obtained solution was lowered to 10-15°C and its pH was adjusted to 6 to 7 with 6 N NaOH, was slowly stirred at 20-40 rpm for 3 hours, and filtered under reduced pressure, and the thus-obtained solid was washed with 2 L of purified water. The resultant was dried under vacuum at 50°C to obtain L-serine with a yield of 91%.

**[Table 5]**

| Grade 1 heavy metal content in Example 3 | | | |
|---|---|---|---|
| | Before purification (a) | After purification with activated carbon (primary, b) | After purification with ion exchange resin (secondary, c) |
| Mercury content in L-serine | 72 ppb | 46 ppb | 37 ppb |
| Lead content in L-serine | 86 ppb | 52 ppb | 32 ppb |
| Cadmium content in L-serine | 69 ppb | 44 ppb | 26 ppb |
| Arsenic content in L-serine | 76 ppb | 55 ppb | 37 ppb |

### Example 4

At room temperature, 4.0 kg of L-serine (Hunan Furui, China) and 20 L of purified water were added to a reactor and the mixture was stirred for 10 minutes. The pH of the mixture was adjusted to about 3.3 to about 3.5 using concentrated sulfuric acid (Daejung Chemicals & Metals) and the complete dissolution was confirmed. After stirring for 1 hour, a celite pad was spread, and 150 g of amorphous activated carbon (Samchun Chemicals) was spread on top of the celite pad, and filtration was performed under reduced pressure, taking care not to damage the layered surface. The content of class 1 heavy metals in the filtered solution was checked using an ICP analyzer. A celite pad was spread and 12 kg of TRILITE^{®} CLR-8 (purchased from Samyang Corporation) was filled on top of the celite, and then the thus-obtained solution was added and filtration was performed under reduced pressure. The ion exchange resin was collected, and the temperature of the thus-obtained solution was lowered to 10-15°C and its pH was adjusted to 6 to 7 with 6 N NaOH, was slowly stirred at 20-40 rpm for 3 hours, and filtered under reduced pressure, and the thus-obtained solid was washed with 2 L of purified water. The resultant was dried under vacuum at 50°C to obtain L-serine with a yield of 80%.

**[Table 6]**

| Grade 1 heavy metal content in Example 4 | | | |
|---|---|---|---|
| | Before purification (a) | After purification with activated carbon (primary, b) | After purification with ion exchange resin (secondary, c) |
| Mercury content in L-serine | 72 ppb | 33 ppb | 19 ppb |
| Lead content in L-serine | 86 ppb | 76 ppb | 47 ppb |
| Cadmium content in L-serine | 69 ppb | 40 ppb | 42 ppb |
| Arsenic content in L-serine | 76 ppb | 51 ppb | 43 ppb |

As shown in the class 1 heavy metal contents of Examples 1 to 4, it can be seen that when both activated carbon and ion exchange resin are used, the class 1 heavy metal content in L-serine is significantly reduced.

Hereinafter, the effect of class 1 heavy metals on neurological diseases or metabolic diseases through experimental examples on L-serine obtained in Example 1.

### Experiment Example 1

HT22 cells were cultured under 37°C and 5% CO₂ conditions using DMEM medium containing 10% FBS, 100 unit/mL penicillin, and 100 µg/mL streptomycin. The cell lines cultured in a 6-well plate were seeded at a concentration of 5 × 10⁵ cells/well and cultured for 20 hours under 37°C and 5% CO₂ conditions, pre-treated with a drug when cell confluency reached 80% and incubated for 4 hours, and treated with 20 µM DMNQ together with the drug, and incubated for 2 hours. After checking the condition of the cells, 5 µM JC-1 was added to the medium and the cells were stained for 30 minutes under 37°C and 5% CO₂ conditions, treated with 0.25% trypsin-EDTA and the cells were collected in an e-tube, washed twice with PBS, and analyzed with Attune NxT.

The effect of L-serine, which contains class 1 heavy metals, on mitochondria, which affects both neurological diseases and metabolic diseases, was confirmed depending on the concentration of class 1 heavy metals. DMNQ causes oxidative stress and excessive production of ROS, which induces mitochondrial membrane potential depolarization, thereby resulting in mitochondrial dysfunction and apoptosis. In mitochondria, an electrochemical proton gradient is created through the mitochondrial membrane, which produces ATP, and when mitochondria are damaged and apoptosis occurs, mitochondrial permeability pores are opened and thus mitochondrial transmembrane potential decreases (membrane potential depolarization). Mitochondrial membrane potential difference, which is an indicator of apoptosis, was measured through JC-1 staining. JC-1 enters the mitochondria in normal cells due to changes in mitochondrial membrane potential and forms a multicomplex, thereby giving it a red color, whereas when apoptosis occurs due to depolarization of the mitochondrial membrane potential, JC-1 remains in the cytoplasm and gives it a green color. Four hours before DMNQ treatment, neurons were treated with unpurified L-serine, primary purified L-serine, and secondary purified L-serine, and then treating with DMNQ for 2 hours and 30 minutes, the degree of protection of neurons was measured by measuring the mitochondrial membrane potential difference through JC-1 staining.

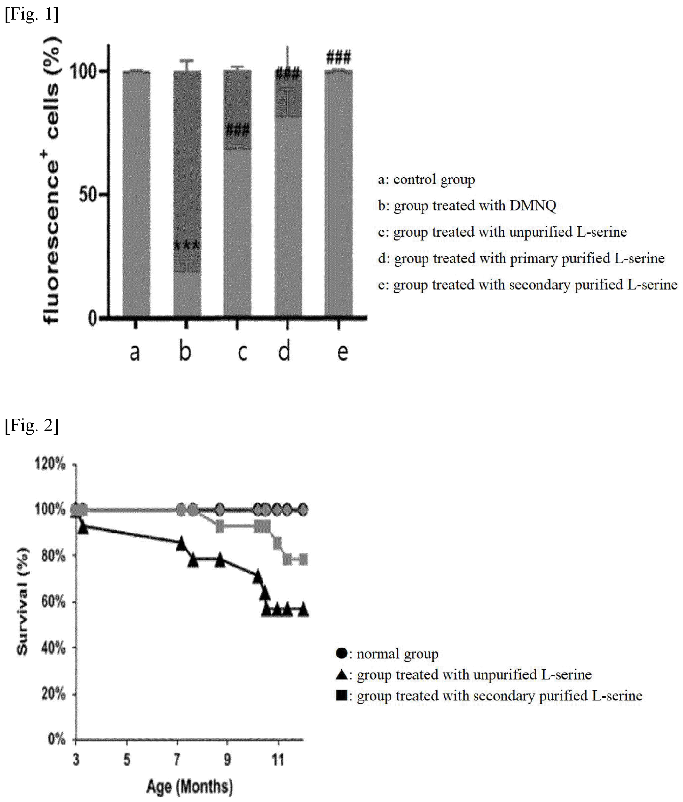

As a result, the groups treated with L-serine showed an inhibitory effect on mitochondrial damage (green: 81.4±4.2%) caused by DMNQ in a manner depending on the content of class 1 heavy metals (green; 0.5 mM: 32.1±1.52%, 1 mM: 18.7±11.1%, 2.5 mM: 1.5±0.23%). In particular, it was confirmed that secondary purified L-serine, which containd all class 1 heavy metals at a concentration below 50 ppb, showed a damage inhibition effect similar to that of the control group (see FIG. 1).

### Experiment Example 2

In order to measure the extent to which the class 1 heavy metal content in L-serine affects neurons when taken over a long period of time, long-term administration of L-serine for 9 months from the early stage of the disease (3 months of age) to 12 months of age was conducted using 5XFAD (Tg6799; five familial mutation), which is an animal model of Alzheimer's disease, and the effect of the drug on animal viability was confirmed. Specifically, WT & 5XFAD mice (purchased from The Jackson Lab) administered with saline and WT & 5XFAD mice administered with L-serine were composed of 12 mice per group. Saline and 500 mg/kg of unpurified L-serine and secondary purified L-serine were orally administered 5 days a week for 9 months, and after administration, the survival rate (%) was measured through daily observation of mice and designated as survival rate.

As a result, it was confirmed that the survival rate of the group treated with secondary purified L-serine was significantly higher than that of the group treated with unpurified L-serine (80% *vs*. 58%) (see FIG. 2).

### Experiment Example 3

The cerebral cortex of a mouse fetus taken from the mother of the mouse was separated, transferred to a HBSS solution, cut into small pieces, and treated with 0.5% trypsin-EDTA in a 37°C water bath for 20 minutes. After adding an equal volume of culture medium, cells were separated by a mechanical method using a pipet, and single cells were separated using a 40 µm cell strainer. After centrifugation at 2,000 rpm for 5 minutes, the supernatant was removed and resuspended in culture medium.

### <Preparation of Plate>

Preparation of PEI solution: A 50% PEI solution, which was prepared by mixing borate buffer and PEI in a 1:1 ratio, was diluted with a 0.1% PEI solution using borate buffer and then filtered using a 0.22 µm filter.

Preparation of Laminin solution: Laminin stock stored at -20°C was thawed at 4°C and then diluted in serum-free medium (20 ng/mL). 40 µL of 0.1% PEI was added to each well of a 24-well MEA plate, and then the 24-well MEA plate was placed in a cell incubator at 37°C and 5% CO₂ for 60 minutes. After washing the 24-well MEA plate three times with sterile DW, the laminin solution was added 50 µL per well and placed overnight in a cell incubator at 37°C and 5% CO₂.

### <Primary cortical neuronal cultures>

After removing the laminin solution from the 24-well MEA plate, the prepared primary neurons were seeded at a concentration of 1.0 × 10⁵ cells/well. Neuronal medium containing 2% B-27 plus supplement, 100 unit/mL penicillin, and 100 µg/mL streptomycin was replaced every 3-4 days up to DIV (day *in vitro*) 14 to 18 and cultured under conditions of 37°C and 5% CO₂. The optimization of neuronal network electrical activity was confirmed at DIV 14-18 using the Maestro Edge system (Axion Biosystems, GA). Neuronal electrical activity was measured using the Maestro Edge system every 30 minutes for 15 minutes, and the same amount of secondary purified L-serine was pretreated for 4 hours based on 0.1 mM unpurified L-serine. After drug pretreatment for 4 hours, neuronal electrical activity was measured 4 times at 30 minute intervals during 2-hour treatment with 20 µM DMNQ, and the data was analyzed using a neural metric tool and an axis metric plotting tool.

A multi-electrode array (MEA) was used to determine how much the class 1 heavy metal content affects the neuroprotective effect of L-serine. For the MEA, neurons were cultured on electrodes for several weeks to form a network and then the electrical signals, that is action potentials, generated from the neurons were measured. This method allows simultaneous measurement of cellular responses to external stimuli or environmental changes from as many cells as there are electrodes, and is a widely used method for drug screening through spontaneous or corresponding voltage changes in response to stimuli or treatment, through action potential recording of the entire neuronal network *in vivo* and *in vitro*.

The primary neuronal culture was cultured in 24-well MEA plates for approximately 15 days, and when electrical signals were passed through sporadic bursts and activated into synchronous network bursts, test substances were treated at concentrations not affecting cell viability, and action potentials were measured and compared.

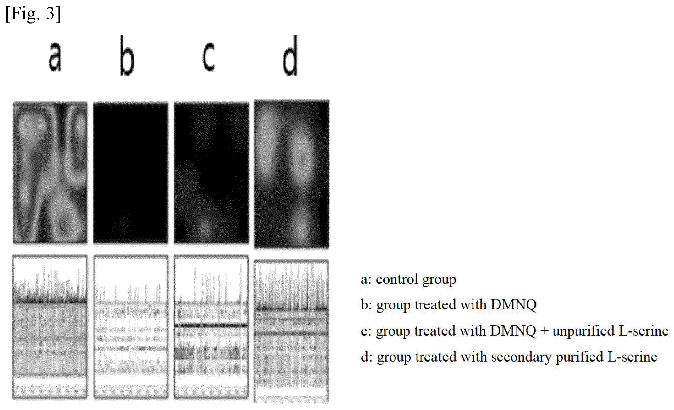

As a result, when primary neurons were treated with DMNQ at a concentration not affecting cell viability (b), active electrodes (spikes and network bursts) were significantly reduced compared to the control group (a). In the case of the group treated with unpurified L-serine (c), there was a change in active electrodes reduced by DMNQ, but the effect was not distinct. In contrast, in the case of the group treated with DMNQ + secondary purified L-serine (d), it was confirmed that active electrodes in a state without DMNQ treatment were maintained even after treatment with DMNQ. From these results, it can be seen that L-serine has a protective effect against neurotoxicity caused by DMNQ, and that the protective effect increases by decreasing the content of class 1 heavy metals in L-serine (see FIG. 3).

As described above, the specific parts of the present invention have been described in detail, and thus it is clear to those skilled in the art that these specific techniques are merely preferred embodiments and will not limit the scope of the present invention. Accordingly, the actual scope of the present invention will be defined by the claims and equivalents thereof.

## Claims

1. A method for preparing L-serine with reduced heavy metal content, comprising:
(a) dissolving L-serine of Formula 1 below in water;
(b) adjusting the pH of the aqueous solution of L-serine obtained in the step (a) with an acid;
(c) passing the pH-adjusted aqueous solution of L-serine through activated carbon and/or ion exchange resin; and
(d) neutralizing the aqueous solution of L-serine obtained in the step (c) while gradually lowering the temperature, 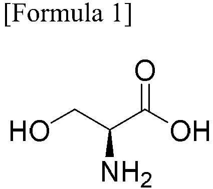 wherein the heavy metal is one or more selected from the group consisting of cadmium, arsenic, lead, and mercury.

2. The method for preparing L-serine of claim 1, wherein heavy metal contents are each 50 ppb or less per gram of L-serine.

3. The method for preparing L-serine of claim 1, wherein in the step (b), the pH is adjusted to 1 to 5.

4. The method for preparing L-serine of claim 1, wherein in the step (b), the pH is adjusted with one or more acids selected from the group consisting of hydrochloric acid, acetic acid, nitric acid, *p*-toluenesulfonic acid, fumaric acid, citric acid, succinic acid, salicylic acid, oxalic acid, bromic acid, phosphoric acid, methanesulfonic acid, tartaric acid, sulfuric acid, and mandelic acid.

5. The method for preparing L-serine of claim 1, wherein in the step (d), the temperature is lowered to 10°C to 15°C.

6. The method for preparing L-serine of claim 1, wherein in the step (d), the neutralization is performed with one or more bases selected from the group consisting of potassium hydroxide, calcium hydroxide, potassium carbonate, calcium carbonate, sodium hydroxide, sodium carbonate, sodium phosphate, and potassium phosphate.

7. The method for preparing L-serine of claim 1, further comprising, after the step (d), (e) performing filtration under reduced pressure and drying the obtained solid.

8. L-serine in which the contents of cadmium, arsenic, lead, and mercury are each 50 ppb or less per gram of L-serine.

9. A pharmaceutical composition for preventing or treating neurological diseases or metabolic diseases comprising the L-serine of claim 8 as an active ingredient.

10. The pharmaceutical composition of claim 9, wherein the neurological disease is selected from the group consisting of developmental disorders, developmental delays, autism spectrum disorder, attention deficit hyperactivity disorder (ADHD), Alzheimer's disease, Parkinson's disease, Huntington's disease, emotional disorders, sleep disorders, and movement disorders.

11. The pharmaceutical composition of claim 9, wherein the metabolic disease is selected from the group consisting of fatty liver, nonalcoholic steatohepatitis, abdominal obesity, hypertriglyceridemia, hypo-HDL-cholesterolemia, hypertension, abnormal glucose metabolism syndrome, type 2 diabetes, cardiovascular disease, myocardial infarction, and angina pectoris.

12. The pharmaceutical composition of claim 9, wherein the content of L-serine in the composition is 60 wt% to 100 wt%.

13. The pharmaceutical composition of claim 9, wherein the composition is administered orally or intravenously.

14. A health functional food composition for preventing or mitigating neurological diseases or metabolic diseases, comprising the L-serine of claim 8 as an active ingredient.

15. The health functional food composition of claim 14, wherein the neurological disease is selected from the group consisting of developmental disorders, developmental delays, cognitive decline, autism spectrum disorder, attention deficit hyperactivity disorder (ADHD), Alzheimer's disease, Parkinson's disease, Huntington's disease, emotional disorders, sleep disorders, and movement disorders.

16. The health functional food composition of claim 14, wherein the metabolic disease is selected from the group consisting of fatty liver, nonalcoholic steatohepatitis, abdominal obesity, hypertriglyceridemia, hypo-HDL-cholesterolemia, hypertension, abnormal glucose metabolism syndrome, type 2 diabetes, cardiovascular disease, myocardial infarction, and angina pectoris.

17. The health functional food composition of claim 14, wherein the content of L-serine in the composition is 60 wt% to 100 wt%.

18. A method for preventing or treating neurological disease or metabolic diseases in a subject, comprising administering the L-serine of claim 8 to the subject.

19. Use of the L-serine of claim 8 for preventing or treating neurological diseases or metabolic diseases.

20. Use of the L-serine of claim 8 in preparation of a drug for preventing or treating neurological diseases or metabolic diseases.
